**Europäisches Patentamt**

**European Patent Office**

**Office européen des brevets**

(11) Veröffentlichungsnummer: **0 012 981**
**B1**

(12) **EUROPÄISCHE PATENTSCHRIFT**

(45) Veröffentlichungstag der Patentschrift:
25.11.81

(21) Anmeldenummer: **79105240.0**

(22) Anmeldetag: **18.12.79**

(51) Int. Cl.³: **C 07 C 127/19**

(54) Verfahren zur Herstellung von 2,4-Dinitrophenyl-harnstoff.

(30) Priorität: **23.12.78 DE 2855882**

(43) Veröffentlichungstag der Anmeldung:
**09.07.80 Patentblatt 80/14**

(45) Bekanntmachung des Hinweises auf die Patenterteilung:
**25.11.81 Patentblatt 81/47**

(84) Benannte Vertragsstaaten:
**BE CH DE FR GB IT**

(56) Entgegenhaltungen:
**BEILSTEINS HANDBUCH DER ORGANISCHEN CHEMIE, Vierte Auflage, Zweites Ergänzungswerk, 12er Band (1950) Berlin DE GIÜA, P. »2,4 Dinitrophenylharnstoff«, Seite 410**

(73) Patentinhaber: **HOECHST AKTIENGESELLSCHAFT, Postfach 80 03 20, D-6230 Frankfurt/Main 80 (DE)**

(72) Erfinder: **Papenfuhs, Theodor, Dr., Heinrich-Bleicher-Strasse 40, D-6000 Frankfurt am Main 50 (DE)**
Erfinder: **Gengnagel, Kurt, Dr., Rossertstrasse 88, D-6239 Kriftel (DE)**

Verfahren zur Herstellung von 2,4-Dinitrophenyl-harnstoff

Die vorliegende Erfindung betrifft ein verbessertes Verfahren zur Herstellung von 2,4-Dinitrophenyl-harnstoff, der als Vorprodukt zur Herstellung von Farbstoffen Verwendung findet.

Es ist bekannt, daß man durch Umsetzung von 2,4-Dinitro-1-chlorbenzol mit Cyanamid in Äthanol 2,4-Dinitrophenylcyanamid erhält, das nach Isolierung durch Erhitzen mit alkoholischer Salzsäure in 2,4-Dinitrophenyl-harnstoff übergeführt werden kann (vgl. J. pr. Ch. 110, 300 + 301, [1925]). Ein derartiges Verfahren ist für eine Durchführung in technischem Maßstab nicht geeignet, da das organische Lösungsmittel aus ökologischen Gründen zurückgewonnen werden muß.

Es wurde nun gefunden, daß man 2,4-Dinitrophenyl-harnstoff durch Umsetzung von 2,4-Dinitro-1-chlorbenzol mit Cyanamid und anschließende saure Hydrolyse des 2,4-Dinitrophenyl-cyanamids technisch in einfacher Weise erhalten kann, wenn man die Umsetzung des 2,4-Dinitro-1-chlorbenzols mit dem Cyanamid und die anschließende Hydrolyse in wäßrigem Medium und ohne Zwischenisolierung des 2,4-Dinitrophenylcyanamids vornimmt.

Die vorliegende Erfindung betrifft somit ein verbessertes Verfahren zur Herstellung von 2,4-Dinitrophenyl-harnstoff durch Umsetzung von 2,4-Dinitro-1-chlorbenzol mit Cyanamid und anschließender saurer Hydrolyse des 2,4-Dinitrophenylcyanamids, das dadurch gekennzeichnet ist, daß man die Umsetzung des 2,4-Dinitro-1-chlorbenzols mit dem Cyanamid und die anschließende Hydrolyse in wäßrigem Medium und ohne Zwischenisolierung des 2,4-Dinitrophenyl-cyanamids durchführt.

Die Umsetzung mit Cyanamid erfolgt vorteilhaft bei Temperaturen von 55 bis 75°C, die anschließende Hydrolyse bei 40 bis 75°C.

Das erfindungsgemäße Verfahren kann beispielsweise im einzelnen folgendermaßen durchgeführt werden:

Man trägt das 2,4-Dinitro-1-chlorbenzol in Wasser ein, wobei man vorzugsweise die 2- bis 5fache Gewichtsmenge an Wasser verwendet; anschließend wird Cyanamid im Überschuß und die wäßrige Lösung eines Alkalihydroxyds, wie Natrium- oder Kaliumhydroxyd, und/oder eines Alkalisalzes einer schwachen Säure, wie Natriumcarbonat, in zum Cyanamid etwa äquivalenten Mengen hinzugegeben. Dieser Reaktionsansatz wird anschließend auf etwa 60 bis 65°C erwärmt, wobei die Reaktion einzusetzen beginnt; man läßt die Temperatur auf etwa 70−75°C ansteigen, wobei man erforderlichenfalls kühlt. Nach Erreichen dieser Temperatur wird die Reaktion bei etwa 60−65°C unter weiterem Rühren weitergeführt; die Reaktionszeit sollte etwa 4−5 Stunden Temperaturabfall betragen.
Sodann wird der Reaktionsansatz unter Rühren in wäßrige, etwa 30%ige Salzsäure gegeben, deren Menge so bemessen ist, daß das Reaktionsgemisch stark sauer ist. Dabei sollte die Temperatur von etwa 70°C nicht überschritten werden. Die saure Lösung wird sodann nochmals bei einer Temperatur von etwa 70 bis 75°C für mehrere Stunden, so etwa für 5 bis 6 Stunden, gerührt und anschließend auf eine Temperatur von unterhalb 10°C, insbesondere auf eine Temperatur von 3−5°C, abgekühlt. Bei dieser niedrigen Temperatur scheidet sich der gebildete 2,4-Dinitrophenylharnstoff ab; zur möglichst vollständigen Gewinnung dieser Endsubstanz läßt man vorteilhaft die Abscheidung bei dieser Temperatur unter Rühren für eine Stunde erfolgen. Der 2,4-Dinitrophenyl-harnstoff kann sodann abgesaugt und bei etwa 60°C getrocknet werden. Man erhält ihn in hoher Ausbeute und guter Reinheit.

Um einen vollständigen Reaktionsumsatz zu gewährleisten, verwendet man Cyanamid im Überschuß, vorzugsweise in der 1,5- bis 2,5molaren, insbesondere in der 1,8- bis 2,3molaren Menge, bezogen auf das 2,4-Dinitro-1-chlorbenzol. Das Alkali dient zur Lösung des Cyanamids und wird deshalb in ca. 1- bis 1,2fach äquivalenter Menge, bezogen auf das Cyanamid, eingesetzt.

2,4-Dinitrophenyl-harnstoff ist bekannt. Er kann beispielsweise als Ausgangsverbindung zur Herstellung von 5-Amino-benzimidazolon dienen, so bei der Herstellung von Azoverbindungen beispielsweise als Diazokomponente (vgl. deutsche Offenlegungsschrift Nr. 1 544 394). 5-Amino-benzimidazolon erhält man aus 2,4-Dinitrophenylharnstoff durch katalytische Reduktion der Nitrogruppen und Ringschluß unter Ammoniakabspaltung gemäß der am gleichen Tage eingereichten europäischen Patentanmeldung 79 105 242.6 entsprechend der deutschen Prioritätsanmeldung P 2 855 883.0 vom 23. Dezember 1978.

Das nachstehende Beispiel dient zur Erläuterung des erfindungsgemäßen Verfahrens. Die Teile sind Gewichtsteile, die Prozentangaben beziehen sich auf Gewichtsprozente, sofern nicht anders vermerkt.

Beispiel

In 600 Teile Wasser von 50 bis 55°C werden 200 Teile 2,4-Dinitro-1-chlorbenzol eingetragen. Nach Zugabe von 160 Teilen einer wäßrigen 50%igen Cyanamidlösung, 229 Teilen einer 33%igen Natronlauge und 40 Teilen wasserfreiem Natriumcarbonat, das in 100 Teilen Wasser gelöst sein kann, wird das so hergestellte Reaktionsgemisch auf 60 bis 65°C erwärmt. Bei Erreichen dieser Temperatur beginnt die Reaktion langsam einzusetzen. Ohne weitere Erwärmung, erforderlichenfalls unter Kühlung, läßt man die Reaktion auf eine Temperatur von etwa 70 bis 75° C ansteigen, hält sie bei dieser Temperatur und läßt sie sodann bei Temperaturabfall unter Außenheizung noch etwa 5 Stunden bei 60 bis 65° C unter Rühren zu Ende bringen.

Die warme, alkalische Suspension wird anschließend in eine Mischung aus 1400 Teilen einer 30%igen Salzsäure und 300 Teilen Wasser von 60 bis 70° C eingetragen. Man erwärmt den Reaktionsansatz auf 70 bis 75° C, rührt bei dieser Temperatur sechs Stunden lang nach, kühlt sodann auf 3 bis 5° C ab und rührt bei dieser Temperatur weiterhin eine Stunde nach. Der abgeschiedene 2,4-Dinitrophenyl-harnstoff wird abgesaugt, trockengesaugt und mit 1500 Teilen kaltem Wasser gewaschen, sodann trockengesaugt und im Umluftschrank bei 60° C getrocknet.

Man erhält 213 Teile (entsprechend 95,4% d. Th.) 2,4-Dinitrophenylharnstoff; er hat einen Schmelzpunkt von 197 bis 199° C.

## Patentansprüche

1. Verfahren zur Herstellung von 2,4-Dinitrophenyl-harnstoff durch Umsetzung von 2,4-Dinitro-1-chlorbenzol mit Cyanamid und anschließende saure Hydrolyse des 2,4-Dinitrophenylcyanamids, dadurch gekennzeichnet, daß man die Umsetzung des 2,4-Dinitro-1-chlorbenzols mit dem Cyanamid und die anschließende Hydrolyse in wäßrigem Medium und ohne Zwischenisolierung des 2,4-Dinitrophenyl-cyanamids durchführt.

2. Verfahren nach Anspruch 1, dadurch gekennzeichnet, daß man die Umsetzung mit dem Cyanamid bei Temperaturen von 55 bis 75° C durchführt.

3. Verfahren nach Anspruch 1 oder 2, dadurch gekennzeichnet, daß man die saure Hydrolyse bei einer Temperatur zwischen 40 bis 75° C durchführt.

4. Verfahren nach Anspruch 1, 2 oder 3, dadurch gekennzeichnet, daß man die Umsetzung des 2,4-Dinitro-1-chlorbenzols mit dem Cyanamid mit der 1,5- bis 2,5molaren Menge an Cyanamid, bezogen auf 2,4-Dinitro-1-chlorbenzol, durchführt.

## Claims

1. Process for the preparation of 2,4-dinitrophenyl urea by reaction of 2,4-dinitro-1-chlorobenzene with cyanamide and subsequent acid hydrolysis of the 2,4-dinitrophenyl cyanamide, characterized by that the reaction of the 2,4-dinitro-1-chlorobenzene with the cyanamide and the subsequent hydrolysis is carried out in an aqueous medium and without intermediate isolation of the 2,4-dinitrophenyl cyanamide.

2. A process according to claim 1, characterized by that the reaction with the cyanamide is carried out at temperatures of from 55 to 75° C.

3. A process according to claim 1 or 2, characterized by that the acid hydrolysis is carried out at a temperature between 40 to 75° C.

4. A process according to claim 1, 2 or 3, characterized by that the reaction of the 2,4-dinitro-1-chlorobenzene with the cyanamide is carried out with the use of 1.5 to 2.5 the molar amount of cyanamide, calculated on 2,4-dinitro-1-chlorobenzene.

## Revendications

1. Procédé de préparation de la dinitro-2,4-phényl-urée par réaction du dinitro-2,4 chloro-1 benzène avec le cyanamide, puis hydrolyse acide du dinitro-2,4 phénylcyanamide, procédé caractérisé en ce qu'on effectue la réaction du dinitro-2,4 chloro-1 benzène avec le cyanamide et l'hydrolyse ultérieure dans un milieu aqueux et sans isoler intermédiairement le dinitro-2,4 phényl-cyanamide.

2. Procédé selon la revendication 1, caractérisé en ce qu'on effectue la réaction avec le cyanamide à des températures de 55 à 75° C.

3. Procédé selon l'une des revendications 1 et 2, caractérisé en ce qu'on effectue l'hydrolyse acide à une température comprise entre 40 et 75° C.

4. Procédé selon l'une quelconque des revendications 1, 2 et 3, caractérisé en ce qu'on effectue la réaction du dinitro-2,4 chloro-1 benzène avec le cyanamide en utilisant ce dernier en une quantité de 1,5 à 2,5 moles pour 1 mole de dinitro-2,4 chloro-1 benzène.